# EUROPÄISCHE PATENTSCHRIFT

(11) **EP 3 049 401 B1**
(45) Veröffentlichungstag und Bekanntmachung des Hinweises auf die Patenterteilung: **07.11.2018**
(21) Anmeldenummer: 14772124.5
(22) Anmeldetag: 23.09.2014
(51) Int. Cl.: C07D 339/06, C07D 339/08

(54) **POLYMER-FIXIERTE DITHIOLAN- BZW. DITHIANDERIVATE**
POLYMER-FIXED DERIVATIVES OF DITHIOLANE OR DITHIANE
DÉRIVÉS DITHIANES ET DITHIOLANES FIXÉS SUR POLYMÈRES

(30) Priorität: 27.09.2013 DE 102013110740
(43) Veröffentlichungstag der Anmeldung: 03.08.2016
(73) Patentinhaber: Karlsruher Institut für Technologie, 76131 Karlsruhe (DE)
(72) Erfinder: JUNG, Nicole, 64319 Pfungstadt (DE); GRÄSSLE, Simone, 76571 Gaggenau (DE); BRÄSE, Stefan, 53842 Troisdorf (DE)
(74) Vertreter: Fitzner, Uwe
(86) Internationale Anmeldenummer: PCT/EP2014/070186
(87) Internationale Veröffentlichungsnummer: WO 2015/044108

(56) Entgegenhaltungen:
- EP-A1- 0 099 329
- EP-A2- 1 057 808
- DE-A1-102006 017 492
- US-A- 4 469 774
- US-A1- 2011 076 620
- C. CHEN ET AL.: "Synthesis and photophysical studies of silylene-spaced divinylarene copolymers. Molecular weight dependent fluorescence of alternating silylene-divinylbenzene copolymers", J. AMER. CHEM. SOC., Bd. 119, Nr. 46, 1997, Seiten 11321-11322, XP002732202,
- Y.-J. CHENG ET AL.: "Intrachain energy transfer in silylene-spaced alternating donor-acceptor divinylarene copolymers", CHEMICAL COMMUNICATIONS, 7. August 2002 (2002-08-07), XP002732203,

## Beschreibung

Die vorliegende Erfindung betrifft an einer festen Phase fixierte Dithiolanylium-Salze und Dithianylium-Salze sowie Yliden-Dithiane und Yliden-Dithiolane, deren Herstellung sowie deren Verwendung.

### Stand der Technik:

Dithiane oder Dithiolane bzw. deren Oxoderivate sind aus dem Stand der Technik bekannt. Beispielsweise werden in der US 4,946,861 Dithianylidene und deren Oxide sowie deren Verwendung als Pharmazeutika beschrieben.
Es ist bekannt, Dithiane oder Dithiolane für die Umsetzung von Ketonen oder Aldehyden zu verwenden. Dies betrifft unter anderem folgende Anwendungen.
1) als Schutzgruppen in Schutzgruppenstrategien
2) als notwendige Umformung in der Umpolungsreaktion
3) als Zwischenprodukt zur weiteren Derivatisierung z.B. Einführung von Fluor.
Die Umformung der Carbonylfunktionalität hin zum Dithiolan und zum Dithian ist eine mit guten bis sehr guten Ausbeuten durchführbare Reaktion, die jedoch in der Regel folgende Nachteile hat:
1) die Reagenzien sind giftig
2) die Reagenzien stinken extremst
3) die Produkte müssen nach der Umsetzung aufwändig aufgereinigt werden
4) viele Reaktionen sind nicht möglich oder verlaufen schlecht, da die Säure in Lösung säurelabile Gruppen zersetzt.
Aus Synthetic Communications, Vol. 34, No. 24, Seiten 4545-4556, 2004 ist es bekannt, 2-[1,3]Dithian-2-Yliden-3-Oxo-Butanamid als Thioactalisierungs-Reagenz in Flüssigphasenreaktionen einzusetzen, wobei die Aufarbeitung der Produkte immer auch eine chromatographische Aufreinigung umfasst.
Mit 2-(1,3-Dithian-2-yliden)malonsäure ist aus Synthetic Communications, Vol. 37:6, Seiten 993-1000, 2007 ein ähnlicher Stoff für die Thioacetalisierung in Flüssigphasenreaktionen bekannt.

Gemeinsam ist dem Stand der Technik, dass die offenbarten Verbindungen ungebunden, d.h. nicht fixiert, vorliegen und die Reaktionen demgemäß vollständig in flüssiger Phase ablaufen. Außerdem werden keine Verbindungen mit dem Grundkörper eines Polymer-gebundenen endo- oder exozyklisch ungesättigten Dithians oder Dithiolans offenbart. Entsprechend treten die genannten Nachteile auf.

### Aufgabe:

Aufgabe der vorliegenden Erfindung ist es, die Nachteile des Stand der Technik zu vermeiden, insbesondere sehr gut handhabbare Reagenzien zur Verfügung zu stellen, die die Nachteile des Standes der Technik nicht aufweisen, und Verfahren zu deren Herstellung sowie deren Verwendung.

Ebenso sollten neue Chemikalien gefunden werden, die mit Hilfe der erfindungsgemäßen Verbindungen erhalten werden können.

Ebenso sollten unter Verwendung der erfindungsgemäßen Verbindungen auch neue Zugangswege zu, insbesondere neuen, Chemikalien gefunden werden.

### Lösung:

Diese Aufgabe wird gelöst durch
Derivate von 1,3-Dithianen, 1,3-Dithiolanen der allgemeinen Formel 1a 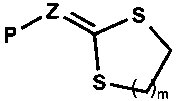 bzw. deren Salzen der allgemeinen Formel 1b 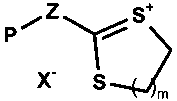 wobei
P = polymerer Träger,
m = 1 oder 2,
   Zausgewählt aus der Gruppe bestehend aus den organischen Resten
   -CH₂-Y-(CH₂)ₙ-CH₂-, -CH₂-Y-(CH₂)ₙ-CH=, -CH₂-Y-CO-(CH₂)ₙ-CH₂-, -CH₂-Y-CO-(CH₂)ₙ-CH=, -CH₂-NH-C₆H₄-Y-(CH₂)ₙ-CH₂- oder -CH₂-NH-C₆H₄-Y-(CH₂)ₙ-CH=, bevorzugt ausgewählt aus der Gruppe bestehend aus den organischen Resten -CH₂-Y-(CH₂)ₙ-CH₂-, -CH₂-Y-(CH₂)ₙ-CH=, -CH₂₋Y-CO-(CH₂)ₙ-CH₂- oder -CH₂-Y-CO-(CH₂)ₙ-CH=
   wobei
   Y = NH oder O,
   n = 0 bis 10, bevorzugt 1 oder 2.
X = anorganisches oder organisches Anion.

### Begriffsdefinitionen:

Im Rahmen der vorliegenden Erfindung sind alle Mengenangaben, sofern nicht anders angegeben, als Gewichtsangaben zu verstehen.

Im Rahmen der vorliegenden Erfindung bedeutet der Begriff "Zimmertemperatur" eine Temperatur von 20 °C. Temperaturangaben sind, soweit nicht anders angegeben, in Grad Celsius (°C).

Sofern nichts anderes angegeben wird, werden die angeführten Reaktionen bzw. Verfahrensschritte bei Normaldruck/Atmosphärendruck, d.h. bei 1013 mbar, durchgeführt.

Im Rahmen der vorliegenden Erfindung schließt die Formulierung "und/oder" sowohl jede beliebige als auch alle Kombinationen der in der jeweiligen Auflistung genannten Elemente ein.

### Detaillierte Beschreibung:

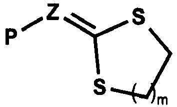

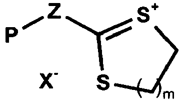

Bevorzugt wird X ausgewählt wird aus der Gruppe bestehend aus den Anionen Br⁻, Cl⁻, Br₃⁻, ClO₄⁻, HCl₂⁻, BF₄⁻, CF₃SO₃⁻, insbesondere BF₄⁻, CF₃SO₃⁻.

Bevorzugt wird P ausgewählt wird aus der Gruppe bestehend aus Polystyrol, Polypropylen, Polyethylen, Polyethylenterephthalat, Silica, Cellulose eingesetzt. Insbesondere bevorzugt ist Polystyrol.

Im Rahmen der vorliegenden Erfindung ist unter dem polymeren Träger P demgemäß das Polymerrückgrat bzw. die polymere Struktur ohne anhängende funktionelle Gruppen zu verstehen.

Selbstverständlich ist es im Rahmen der vorliegenden Erfindung auch möglich, mehrere verschiedene polymere Träger in Mischung einzusetzen.
Ebenso ist es im Rahmen der vorliegenden Erfindung möglich, verschiedene Gruppen Z (Linkergruppen) in Mischung zu verwenden, gegebenenfalls auch zusammen mit Mischungen von polymeren Trägern P.

Bevorzugte erfindungsgemäße Verbindungen sind solche ausgewählt aus der Gruppe bestehend aus den Verbindungen der folgenden Formeln 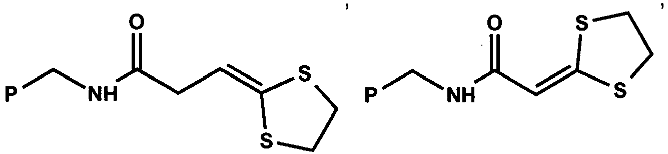 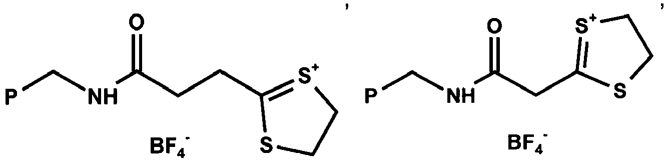 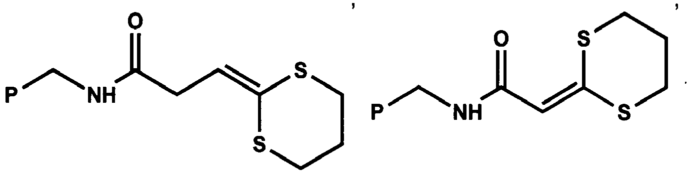 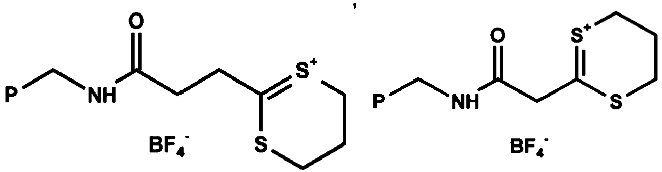 und Mischungen davon.

Besonders bevorzugte Verbindungen sind solche der Formel 1a, in denen P = Polystyrol, m=2, Z=-CH₂-Y-CO-(CH₂)ₙ-CH= mit Y= NH,
und solche Salze der Formel 1b, in denen
P = Polystyrol, m=2, Z=-CH₂-Y-CO-(CH₂)ₙ-CH₂- mit Y= NH und X = BF₄⁻ sind, wobei n wie oben definiert ist.

Selbstverständlich umfasst die vorliegende Erfindung auch Varianten, bei denen der Linker Z an den polymeren Träger P, anstelle der in den Formeln dargestellten Einfachbindung, über Doppel- oder Dreifachbindungen gebunden ist. Weiterhin umfasst die vorliegende Erfindung selbstverständlich Produkte, die eine Vielzahl von Linker/Dithiolan-/Ditianeinheiten an einem polymeren Träger aufweisen; dies ist bei der formelmäßigen Darstellung der Einfachheit und besseren Lesbarkeit halber nicht dargestellt. Entsprechend können an einem polymeren Träger auch verschiedene Linker Z über Einfach-, Doppel- und/oder Dreifachbindungen gebunden sein.

Gegenstand der vorliegenden Erfindung ist auch ein Verfahren zur Herstellung der erfindungsgemäßen Verbindungen.
Die vorliegende Erfindung stellt dabei eine einfache Synthese der erfindungsgemäßen Verbindungen zur Verfügung, wobei ein festes Trägermaterial eingesetzt wird, das dann durch Modifikation sowohl die Thioeinheit als auch - falls gewünscht - Säure enthält.

Gegenstand der vorliegenden Erfindung ist mithin auch ein Verfahren zur Herstellung der erfindungsgemäßen Verbindungen umfassend die Verfahrensschritte oder bestehend aus den Verfahrensschritten
a) Bereitstellen mindestens eines polymeren Trägers mit anhängigen funktionellen Gruppen
b) Umsetzung der funktionellen Gruppen des/der polymeren Träger(s) mit mindestens einem Kettenverlängerungsmittel,
c) gegebenenfalls Modifikation der funktionellen Gruppen des aus b) resultierenden kettenverlängerten Produktes,
d) Umsetzung des in b) bzw. c) erhaltenen Produktes mit gegebenenfalls substituierten aliphatischen oder aromatischen Dithioverbindungen mit zwei oder drei Kohlenstoffatomen zwischen den Schwefelatomen, bevorzugt aliphatischen Dithioverbindungen, insbesondere bevorzugt 1,2-Dithioethan oder 1,3-Dithiopropan,
e) optional Aufarbeitung des in Schritt d) erhaltenen Produktes
   e1) zu den entsprechenden Dithiolanylium-Salzen/Dithianylium-Salzen (1b), bevorzugt durch Waschen unter Verwendung von Diethylether und THF,
      oder
   e2) zu Yliden-Dithiolanen/Dithianen (1a), bevorzugt durch Waschen mit anderen Lösemitteln als Diethylether/THF oder durch Zusatz von Base im Waschlösemittel.

Zur Herstellung der erfindungsgemäßen Verbindungen geht man im allgemeinen wie folgt vor:
Zunächst wird mindestens ein polymerer Träger, bevorzugt ein polymerer Träger, zur Verfügung gestellt, der anhängende funktionelle Gruppen aufweist, über die weitere Reste addiert werden können.
Sofern der polymere Träger die funktionellen Gruppen nicht von vorneherein aufweist, kann der polymere Träger durch fachübliche Maßnahmen funktionalisiert werden.
Die funktionellen Gruppen können beispielsweise Hydroxylgruppen (insbesondere bei Silica als Träger) oder Aminogruppen (insbesondere bei organischen Polymeren wie Polystyrol als Träger) sein. Genauso können dies aber auch Halogene oder Carbonyle im weiteren Sinne (Aldehyde/Ketone/Carbonsäuren oder Carbonsäurechloride) o.ä. sein.
Danach erfolgt eine Kettenverlängerung über Reaktion mit den funktionellen Gruppen des Polymers mit funktionellen Gruppen einer anderen Verbindung. Dabei muss allerdings sichergestellt sein, dass am Ende des Moleküls eine funktionelle Gruppe verbleibt. Dafür eigenen sich zum Beispiel Carbonsäureanhydride oder Dicarbonsäuren gut, wodurch eine Carbonsäuregruppe verbleibt. Weiterhin können auch Dicarbonsäurechloride, Diamine, Diole o.ä eingesetzt werden.
Gegebenenfalls kann dabei eine katalytisch wirksame Verbindung zugesetzt werden.
Die funktionelle Gruppe, welche am vom polymeren Träger abgewandten Ende verbleibt, kann gegebenenfalls modifiziert werden, um für den nachfolgenden Reaktionsschritt besser geeignet zu sein. Im Falle einer Carbonsäuregruppe kann dies zum Beispiel die Modifikation mit Thionylchlorid zur Säurechloridgruppe sein.
In dem nachfolgenden Reaktionsschritt (also nach Kettenverlängerung und optionaler Modifikation) wird mit einer Dithioverbindung, bevorzugt 1,2-Dithioethan oder 1,3-Dithiopropan, umgesetzt, wodurch je nach Reaktionsbedingungen die erfindungsgemäßen, an den polymeren Träger gebundenen, 1,3-Dithian-2-ylidene und 1,3-Dithiolan-2-ylidene oder die entsprechenden Dithianylium- und Dithiolanylium-Salze resultieren.
In diesem Schritt ist es auch möglich andere Dithioverbindungen einzusetzen, sofern diese zwischen den beiden Schwefelatomen zwei bzw. drei Kohlenstoffatome aufweisen. Beispielsweise 1,2-Dithiopropan, 1,3-Dithiobutan oder ähnliche Verbindungen.

Vorteilhaft kann man im Rahmen der vorliegenden Erfindung auch von bereits funktionalisierten polymeren Trägern ausgehen, da diese oftmals käuflich erhältlich sind. Ein bevorzugter bereits funktionalisierter polymerer Träger, von dem im Rahmen der vorliegenden Erfindung ausgegangen werden kann, ist Polystyrol-(CH₂NH₂)ₓ, wobei x die Anzahl der anhängenden Aminomethylengruppen darstellt. Selbstverständlich sind auch andere entsprechende Verbindungen einsetzbar, sofern diese entsprechende Funktionalisierungen aufweisen, wie z.B. Polystyrol-(CH₂Cl)ₓ.

Insbesondere bevorzugt ist erfindungsgemäß ein Verfahren, umfassend oder bestehend aus den folgenden Verfahrensschritten:
a) Bereitstellen von Polystyrol mit anhängigen -CH₂-NH₂-Gruppen,
b) Umsetzung mit Bernsteinsäureanhydrid in Dimethylformamid (DMF) und/oder Triethylamin, optional in Gegenwart von 4-(Dimethylamino)-pyridin (DMAP),
c) Umsetzung der resultierenden Carbonsäure mit Thionylchlorid zum Säurechlorid,
d) Umsetzung mit 1,2-Dithioethan oder 1,3-Dithiopropan in Gegenwart von HBF₄ in Diethylether
e) optional Aufarbeitung des in Schritt d) erhaltenen Produktes durch Entfernen des Lösemittels und Waschen des zurückbleibenden Harzes, bevorzugt mit Diethylether und/oder THF und anschließendes Trocknen des Harzes.
In einer Variante ist es bevorzugt, die Umsetzung in Schritt b) in Gegenwart von DMAP durchzuführen.

Eine weiteres besonders bevorzugtes erfindungsgemäßes Verfahren, umfasst oder besteht aus den folgenden Verfahrensschritten:
a) Bereitstellen von Polystyrol mit anhängigen -CH₂-NH₂-Gruppen,
b) Umsetzung mit Adipoyldichlorid in Triethylamin, optional in Gegenwart von 4-(Dimethylamino)-pyridin (DMAP),
d) Umsetzung mit 1,2-Dithioethan oder 1,3-Dithiopropan in Gegenwart von HBF₄ in Diethylether
e) optional Aufarbeitung des in Schritt d) erhaltenen Produktes durch Entfernen des Lösemittels und Waschen des zurückbleibenden Harzes, bevorzugt mit Diethylether und/oder THF und anschließendes Trocknen des Harzes.
In einer Variante ist es bevorzugt, die Umsetzung in Schritt b) in Gegenwart von DMAP durchzuführen.

Die bevorzugte Reaktion eines Carbonsäureanhydrids mit einer dem polymeren Träger anhängigen Amingruppe (z.B. als Aminomethylengruppe) ist besonders vorteilhaft, weil sie zum einen die Anbindung der Schwefelgruppierung ermöglicht und zum anderen der Linker bzw. das angebundene Derivat auch bei der sauren Chlorierungsreaktion und der unter sauren Bedingungen durchgeführten Salzbildung stabil bleibt.

Die Aufarbeitung des in Schritt d) erhaltenen Produktes kann durch Wahl der Lösemittel so optimiert werden, dass das Salz an der festen Phase erhalten bleibt. Salze am polymeren Träger sind selten und auch schwierig über die Waschschritte zu erhalten.
Insbesondere kann man im Rahmen der vorliegenden Erfindung wie folgt vorgehen: die Reaktionsmischung wird in eine Fritte überführt, das Lösemittel wird über Filtration entfernt und das zurückbleibende Harz wird mit Lösemittel gewaschen, bis es geruchsneutral ist. Besonders bevorzugt sind hierbei Diethylther und THF (im Gegensatz zu anderen Lösemitteln erhalten diese Lösungsmittel das Salz am Harz intakt). Das Harz wird dann im Hochvakuum getrocknet.
Entsprechend ist es eine bevorzugte Variante der vorliegenden Erfindung das der Aufarbeitungsschritt e) obligatorisch ist.

Schließlich sind Gegenstände der vorliegenden Erfindung die verschiedenen Verwendungen der erfindungsgemäßen Verbindungen bzw. der nach dem erfindungsgemäßen Verfahren hergestellten Verbindungen.

Die erfindungsgemäßen Verbindungen können auch als Dithioalkyl-Reagenzien bezeichnet bzw. verwendet werden.
Die erfindungsgemäßen Verbindungen sind Dithianylium- und Dithiolanylium Salze sowie 1,3-Dithian- oder 1,3-Dithiolan-2-ylidene.

Die erfindungsgemäß eingesetzten Linker Z dienen der Anbindung der Thiokomponenten an den polymeren Träger. Bevorzugt weisen sie zur Anbindung an den polymeren Träger Strukturen auf, die bei der Herstellung der erfindungsgemäßen Verbindungen während der sauren Chlorierungsreaktion und der unter sauren Bedingungen durchgeführten Salzbildung stabil bleiben.

Bevorzugt werden dafür Linker Z eingesetzt, die über eine C-C oder eine C-N-Verknüpfung an den polymeren Träger gebunden sind. Sehr gut geeignet sind hierfür Amidgruppen.
Besonders bevorzugt sind erfindungsgemäß die oben angegebenen Linker Z.

Selbstverständlich können im Rahmen der vorliegenden Erfindung die Linker Z durch Umsetzung mit verschiedenen Stoffen erhalten werden. Beispielsweise auch durch Umsetzung mit Aminosäuren (wie beta- oder gamma-Aminosäuren).

Mittels der erfindungsgemäßen Verbindungen lassen sich im Bereich der organischen Chemie beispielsweise bei der Katalyse und auch für stöchiometrische Umsetzungen hervorragende Ergebnisse erzielen, so lassen sich Schutzgruppen besonders leicht einführen und Reaktionen mit HBF₄ besonders einfach und ökonomisch durchführen.

Die erfindungsgemäßen Verbindungen weisen folgende Vorteile auf:
1) Die erfindungsgemäßen Verbindungen/Reagenzien sind durch feste Anbindung an das Trägermaterial nicht giftig und einfach zu händeln/zu dosieren
2) Die erfindungsgemäßen Verbindungen/Reagenzien weisen aufgrund der Immobilisierung an dem polymeren Träger so gut wie keine Geruchsemissionen auf, sondern sind (vollkommen) geruchsneutral
3) Ein sehr hoher Prozentsatz der mit den erfindungsgemäßen Verbindungen/Reagenzien durchgeführten Reaktionen muss nach der Umsetzung keinen aufwändigen Aufreinigungsschritt umfassen. Es ist keine Chromatographie notwendig; in den meisten Fällen genügt einfaches Abfiltrieren des Polymers und Einrotieren der Reaktionsmischung.
   Die hohe Reinheit der mittels der erfindungsgemäßen Verbindungen/Reagenzien erhaltenen Produkte basiert auf den besonderen Eigenschaften der vorliegenden Erfindung im Vergleich zum Stand der Technik:
   Nach dem bisherigen Stand der Technik verlaufen Umsetzungen in flüssiger Phase entweder durch die stöchiometrische Zugabe der Reagenzien meist nicht ganz vollständig und beinhalten nach der Reaktion noch etwas Startmaterial, oder bei Zugabe im Überschuss noch Reagenz, das entfernt werden muss. Auch die katalytisch zugegebene Säure ist noch in der Rohmischung vorhanden.
   Bei den erfindungsgemäßen Verbindungen/Reagenzien können sowohl die Thiokomponente (an polymerem Träger gebunden) als auch die Säure (auch an polymerem Träger gebunden) im Überschuss zugegeben werden. Dadurch wird die vollständige Umsetzung gefördert. Der Überschuss führt im Gegensatz zum Stand der Technik allerdings nicht zur Verunreinigung der Mischung, da erst bei Angriff des Carbonyls beide Komponenten vom polymeren Träger gespalten werden.
   Die während der Reaktion frei werdende Säure (z.B. HBF₄) bleibt nach beendeter Reaktion immobilisiert am Trägermaterial zurück und gelangt auf diese Weise nicht in die Produktmischung.
4) Mit den erfindungsgemäßen Verbindungen ist es möglich, Reaktionen durchzuführen, bei denen durch Zugabe von Basen in der flüssigen Phase (z.B. Carbonate) eine basische Umgebung generiert wird, während die erfindungsgemäßen Verbindungen 1b am Träger zumindest zum Teil erhalten bleiben. So können auch Verbindungen, die säurelabile Gruppen enthalten, in das Dithiolan/Dithian überführt werden.
5) Die erfindungsgemäßen Verbindungen/Reagenzien sind einfach und kostengünstig herzustellen.
6) Mit den erfindungsgemäßen Verbindungen/Reagenzien durchgeführte Reaktionen führen zu sehr guten Ausbeuten, die in den meisten Fällen höher sind als bei den Reaktionen des Standes der Technik.

Die besonderen Vorteile der vorliegenden Erfindung werden dabei dadurch erreicht, dass im Gegensatz zum Stand der Technik die Reaktionen mit den erfindungsgemäßen Verbindungen/Reagenzien keine reinen FlüssigphasenReaktionen sind, sondern aufgrund der Fixierung der Thiokomponente über eine Linkergruppe an einem polymeren Träger eine feste Reagenz-Phase entsteht.

Mithin ist es ein wesentlicher Aspekt der vorliegenden Erfindung, dass die erfindungsgemäßen Verbindungen als Festphasenreagenzien verwendet werden.

Bei den erfindungsgemäßen Verbindungen konnte überraschenderweise ein Mittelweg zwischen sehr stabiler Anbindung an das Harz (gut, da geruchsfrei, leicht herzustellen und stabil) und leichter Übertragung des Thiolteils (gute und schnelle Reaktionsergebnisse) gefunden werden.

Ein weiterer Vorteil der erfindungsgemäßen Verbindungen ist es, dass bei deren Verwendung zur Einführung von Thioacetalschutzgruppen in Aldehyde/Ketone die Reaktion nicht wasser-sensitiv ist, sondern dass (geringe) Mengen Wasser toleriert werden; die Apparaturen müssen also nicht vorher getrocknet werden. Zudem können die Reaktionen in normaler Atmosphäre durchgeführt werden, die Gegenwart von Inertgas ist nicht notwendig.

Die erfindungsgemäßen Verbindungen können verwendet werden, um sowohl Aldehyde als auch Ketone derart umzusetzen, dass eine Thioacetal- bzw. Ketalgruppe als Schutzgruppe eingeführt wird.
Bei der Umsetzung von Aldehyden sind keine weiteren Additive notwendig.
Bei der Umsetzung von Ketonen ist es unter Umständen vorteilhaft und daher bevorzugt, eine Säure zuzusetzen. Bevorzugt werden hier Lewis-Säuren eingesetzt, die auch für die Flüssigphasen-Umsetzung von Ketonen zu Dithianen verwendet werden, wie beispielsweise ZnCl₂, NiCl₂, CuCl₂, etc.
Ein bevorzugtes Beispiel für eine einsetzbare Säure ist BF₃, die normalerweise in Diethylether eingesetzt wird.

Im Rahmen der vorliegenden Erfindung ist es erstmals möglich, Lewis-Säuren in Kombination mit Thiokomponenten zu immobilisieren.

Die Reaktionen zur Bildung von Dithianen/Dithiolanen verlaufen im allgemeinen in einem Zeitraum zwischen 1 Stunde und 3 Tagen, wobei die genaue Reaktionsdauer selbstverständlich abhängig von den jeweiligen Reaktanten und den Reaktionsbedingungen wie Temperatur und Druck ist.

Erfindungsgemäß ist es möglich, die Reaktionen durch Einwirkung von Mikrowellenstrahlung zu beschleunigen, so dass dann die Reaktionen in weniger als 4 Stunden abgeschlossen werden können.

Sofern Ketone und Aldehyde gleichzeitig vorliegen und keine zusätzliche Säure zugegeben wird, ist die Reaktion der Schutzgruppeneinführung durch die erfindungsgemäßen Verbindungen chemoselektiv für die Aldehyde.

Die Umsetzung der Aldehyde und/oder Ketone läuft erfindungsgemäß bevorzugt in organischem Lösemittel ab. Bevorzugt werden diese ausgewählt aus der Gruppe bestehend aus Acetonitril (MeCN), Dichlormethan, Trichlormethan, Dimethylformamid (DMF), Toluol, Tetrahydrofuran (THF), Methanol, Ethanol und Mischungen davon. Insbesondere bevorzugt ist MeCN, mit diesem werden die saubersten Ergebnisse erzielt, was einen erheblichen Vorteil hinsichtlich der Kosten und bei der Aufarbeitung bedeutet.

Es ist bevorzugt, die erfindungsgemäßen Verbindungen bei deren Verwendung zur Einführung von Schutzgruppen für Aldehyde/Ketone in Form der Salze gemäß Formel 1b einzusetzen.

Allgemein lässt sich die Umsetzung der Aldehyde oder Ketone (die Einführung einer Schutzgruppe in Aldehyde oder Ketone) im Rahmen der vorliegenden Erfindung wie folgt beschreiben:
Aldehyd oder Keton wird in Lösemittel gelöst und eine erfindungsgemäße Verbindung zugegeben. Bei der Umsetzung von Ketonen wird zusätzlich eine Säure, bevorzugt BF₃ in Diethylether, zugegeben. Die Mischung wird erwärmt, bevorzugt bis zum Rückfluss, und für einige Zeit, beispielsweise für 5 Stunden, gerührt oder geschüttelt (in Kolben oder geschlossenem Glasvial), bis vollständige Umsetzung des Ausgangsmaterials, z.B. nachgewiesen per Dünnschichtchromatographie, erreicht ist.

Das Produkt kann dann wie üblich aufgearbeitet werden. Wobei erfindungsgemäß die folgenden drei Varianten bevorzugt werden:
a) Das rohe Reaktionsprodukt wird auf eine Fritte gegeben, wobei der polymere Träger abfiltriert wird. Die Filtrate werden dann z.B. gesammelt und das Lösemittel abgetrennt, z.B. durch Rotationsverdampfer.
b) Das rohe Reaktionsprodukt wird in einen Scheidetrichter enthaltend eine Mischung aus organischem Lösemittel und Wasser, bevorzugt Ethylacetat und Wasser (1:1), gefüllt. Die verschiedenen Lagen werden getrennt und die organische Phase mit Wasser gewaschen (z.B. dreimal). Die organische Phase wird getrocknet, z.B. mit Natriumsulfat, und das Lösemittel abgetrennt, z.B. durch Rotationsverdampfer.
c) Das rohe Reaktionsprodukt wird in einen Scheidetrichter enthaltend eine Mischung aus organischem Lösemittel und Wasser, bevorzugt Ethylacetat und Wasser (1:1), gefüllt. Die verschiedenen Phasen werden getrennt und die organische Phase mit Wasser gewaschen (z.B. dreimal). Die organische Phase wird getrocknet, z.B. mit Natriumsulfat, und die Feststoffe werden mittels Filtration abgetrennt. Danach wird Silicagel zugegeben. Das rohe Produkt wird dann mittels Chromatographie (z.B. Säulenchromatografie mit Hexan/Ethylacetat in verschiedenen Verhältnissen als Laufmittel) aufgereinigt.

Durch diese Umsetzungen wurden die folgenden neuen Substanzen synthetisiert:
- 2-(1,3-Dithian-2-yl)benzen-1,3-diol,
- 3',4'-Dihydro-1'H-spiro[[1,3]dithian-2,2'-naphthalen],
- 1-Amino-2-(1,3-dithian-2-yl)anthracen-9,10(4aH,9aH)-dion,
- (E)-Ethyl-3-(1,3-dithiolan-2-yl)acrylat,
- (E)-Ethyl 3-(1,3-dithian-2-yl)acrylat
diese sind mithin auch Gegenstand der vorliegenden Erfindung.

Die erfindungsgemäßen Verbindungen sind, insbesondere bei Verwendung als Reagenzien, aufgrund der Fixierung der Dithian- bzw. Dithiolan-Gruppierungen an einem Polymer flexibler einsetzbar als die Verbindungen des Standes der Technik und die Möglichkeiten in verschiedensten Reaktionen extrem vielfältig z.B. zur in situ Fluorierung als Dithianen.

Die erfindungsgemäßen Verbindungen enthaltend gebundene Säure können auch für Katalysezwecke verwendet werden. Dies gilt auch für solche Reaktionen, die unabhängig von der Thiokomponente der erfindungsgemäßen Komponente ablaufen.

Weitere Verwendungen der erfindungsgemäßen Verbindungen sind (exemplarische Darstellungen, wobei die Reaktion jeweils in Lösemittel, bevorzugt Acetonitril, abläuft und andere erfindungsgemäße Verbindungen auch einsetzbar sind):
1) Herstellung von Boranilen, die so nicht bekannt ist. Deren Anwendung als Fluoreszenzmarker ist von Bedeutung. Folgend drei Beispiele: 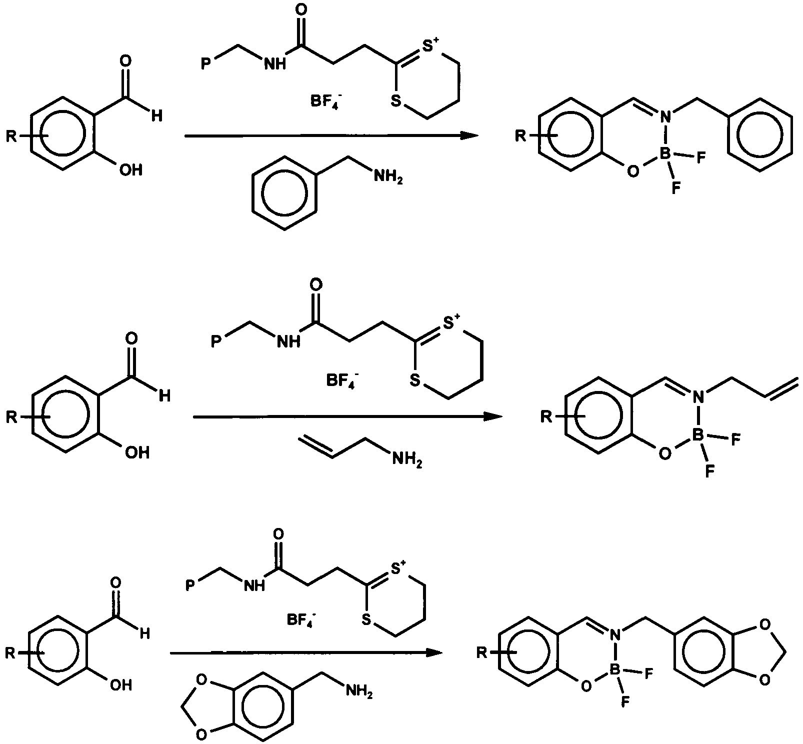
2) Umsetzung von 1,3-Diketoverbindungen zu stabilen BF₂-Addukten, die sehr stark fluoreszieren. 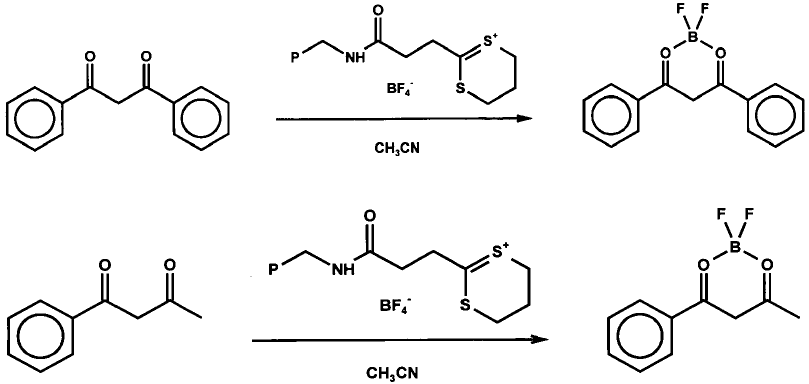
3) Herstellung von Benzimidazolen unter Zugabe von Diaminobenzolen: 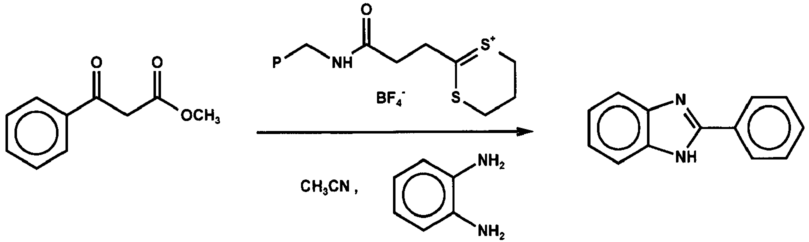
4) Entschützung von mit BOC, THP, Silylschutzgruppen geschützten Alkoholen oder Aminen

Eine weitere mögliche Verwendung der erfindungsgemäßen Verbindungen ist deren Einsatz als lonentauscher, wobei insbesondere die Verbindungen der Formeln 1b zum Einsatz kommen.

Die verschiedenen Ausgestaltungen der vorliegenden Erfindung, z.B. aber nicht ausschließlich diejenigen der verschiedenen abhängigen Ansprüche, können dabei in beliebiger Art und Weise miteinander kombiniert werden.

Die Erfindung wird nun unter Bezugnahme auf die folgenden nicht-limitierenden Beispiele erläutert.

### Beispiele:

### Beispiel 1: Herstellung der Verbindungen gemäß allgemeiner Formel 1a bzw. deren Salzen gemäß allgemeiner Formel 1b.

### Synthese erste Variante

Aminomethylpolystyrolharz (1,00 g; 1,34 mmol) wurde in Chloroform (10 ml) quellen gelassen und mit Bernsteinsäureanhydrid (4,02 mmol; 3,00 Äquiv.), Triethylamin (4,02 mmol; 3,00 Äquiv.) und DMAP (0,67 mmol; 0,50 Äquiv.) versetzt. Das Harz wurde über Nacht bei 80 °C geschüttelt. Dabei wurde das Harz dunkelbraun. Zur Aufarbeitung wurde das Harz mit Aceton (20 ml), Wasser (20 ml), Aceton (20 ml), Methanol (20 ml), Aceton (20 ml) und Dichlormethan (2x20 ml) gewaschen und zunächst im Trockenschrank, dann im Hochvakuum getrocknet.
Das Produkt (1,00 g; 1,18 mmol) wurde dann mit Thionylchlorid (10 ml) versetzt. Das Harz wurde bei 40 °C über Nacht geschüttelt. Zur Aufarbeitung wurde das Harz mit trockenem Diethylether (3x30 ml) gewaschen und im Hochvakuum getrocknet.
Das resultierende Harz (1,02 g; 1,18 mmol) wurde unter Argonatmosphäre in abs. Diethylether (10 ml) quellen gelassen und mit Propandithiol (1,5 ml) und HBF₄ in Diethylether (1,5 ml) versetzt. Die Reaktion wurde über Nacht bei 50 °C geschüttelt. Dabei wurde das Harz grün. Zur Aufarbeitung wurde das Harz mit Diethylether (20 ml), THF (2x20 ml) und wieder mit Ether (20 ml) gewaschen und im Hochvakuum getrocknet.

Der Syntheseweg der ersten Variante lässt sich formelmäßig exemplarisch wie folgt darstellen (o = 0 oder 1):

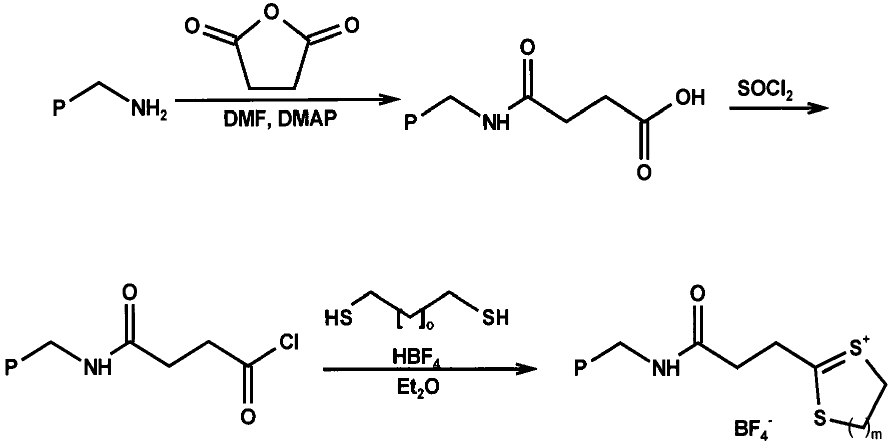

### Synthese zweite Variante

Aminomethylpolystyrolharz (1,00 g; 1,34 mmol) wurde in Chloroform (10 ml) quellen gelassen und mit Adipoyldichlorid (6,70 mmol; 5,00 Äquiv.), Triethylamin (3,620 mmol; 2,70 Äquiv.) und DMAP (0,67 mmol; 0,50 Äquiv.) versetzt. Das Harz wurde über Nacht bei 50 °C geschüttelt. Dabei wurde das Harz braun. Zur Aufarbeitung wurde das Harz mit trockenem Diethylether (100 ml) gewaschen und im Hochvakuum getrocknet.

Das Produkt (1,196 g; 1,334 mmol) wurde unter Argonatmosphäre in abs. Diethylether (10 ml) quellen gelassen und mit Propandithiol (1,5 ml) und HBF₄ in Diethylether (1,5 ml) versetzt. Die Reaktion wurde über Nacht bei 50 °C geschüttelt. Dabei wurde das Harz grün. Zur Aufarbeitung wurde das Harz mit Diethylether (20 ml), THF (2x20 ml) und wieder mit trockenem Diethylether (20 ml) gewaschen und im Hochvakuum getrocknet.

### Beispiel 2: Methode zur Umsetzung von Aldehyden:

Zu 0,1 mmol der jeweiligen Aldehydverbindungen 1, 3 und 5 wurden 200 mg polymeres Trägermaterial (Verbindung A) gegeben und beides in 2 ml MeCN suspendiert. Die Reaktion wurde entweder auf 80 °C oder in der Mikrowelle (100 °C, 1-3 bar, 50-200 Watt) erwärmt.
Nach beendeter Reaktion wurde das Polymer über Filtration und Nachwaschen mit Aceton von der Reaktionsmischung abgetrennt, das Filtrat am Rotationsverdampfer eingeengt und der Rückstand getrocknet. Die Substanz wurde über ¹H, ¹³C NMR auf ihre Reinheit überprüft. Die Ausbeuten lagen für die Verbindungen 1 und 3 bei 95-100% und für Verbindung 5 bei 80-85%.

Es wurden entsprechend die folgenden Verbindungen, aufgeteilt in Gruppen, umgesetzt; deren Charakterisierung erfolgte per Massenspektrometrie bzw. NMR-Spektroskopie und die Reinigung per Chromatographie:
- Gruppe 1: aromatische Aldehyde 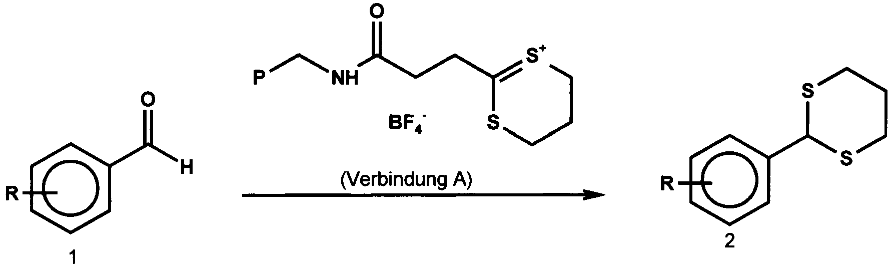 mit R = OMe, OH, Ph, OAllyl, CN, C₆H₅CH₂O, H
- Gruppe 2: aliphatische Aldehyde 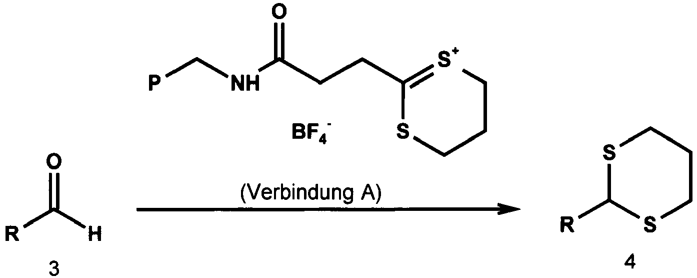 mit R = Me, Indyl, Hexyl
- Gruppe 3: vinylische Aldehyde 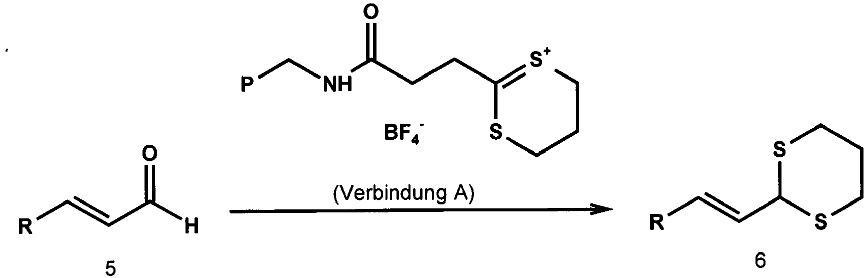 mit R = H, C₆H₄OMe

### Beispiel 3: Methode zur Umsetzung von Ketonen:

Zu 0,1 mmol der jeweiligen Ketoverbindung wurden 200 mg polymeres Trägermaterial gegeben und beides in 2 ml MeCN suspendiert. Es wurde 1 Tropfen BF₃*Et₂O zugegeben. Die Reaktion wurde entweder auf 80 °C oder in der Mikrowelle (100 °C, 1-3 bar, 50-200 Watt) erwärmt.

Nach beendeter Reaktion wurde das Polymer über Filtration und Nachwaschen mit Aceton von der Reaktionsmischung abgetrennt, das Filtrat am Rotationsverdampfer eingeengt und der Rückstand getrocknet. Die Substanz wurde über ¹H, ¹³C NMR auf ihre Reinheit überprüft.

Es wurden entsprechend die folgenden Verbindungen umgesetzt; deren Charakterisierung erfolgte per Massenspektrometrie bzw. NMR-Spektroskopie und die Reinigung (falls erforderlich) per Chromatographie:

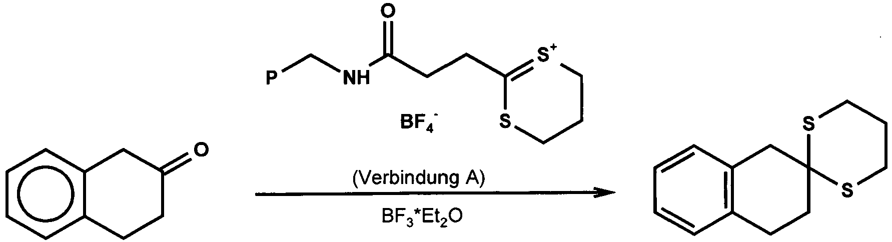

Die Ausbeuten lagen bei 95-100%.

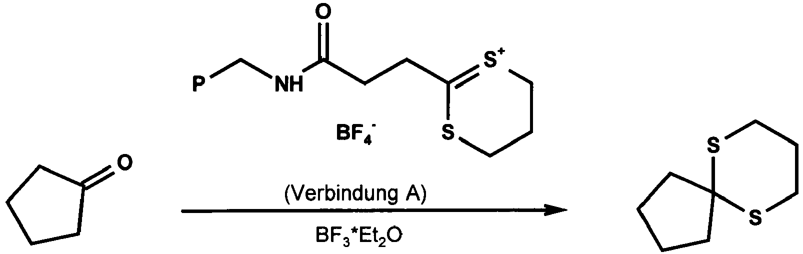

Die Ausbeute lag bei 95-100%. 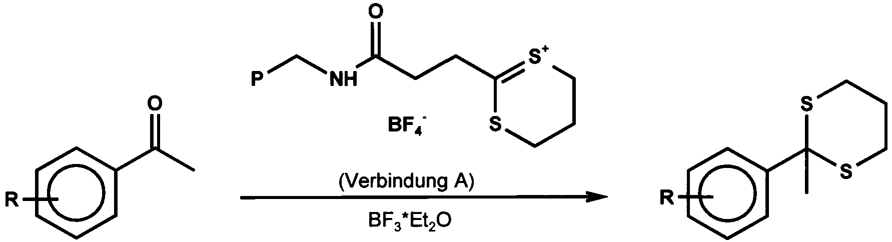 mit R = OMe, Ph

Die Ausbeute lag bei 60%.

### Beispiel 4: Chemoselektivität:

Zur Überprüfung der Chemoselektivität wurde die folgende Reaktion gemäß obiger Vorschrift zur Umsetzung der Aldehyde (Beispiel 2) durchgeführt.

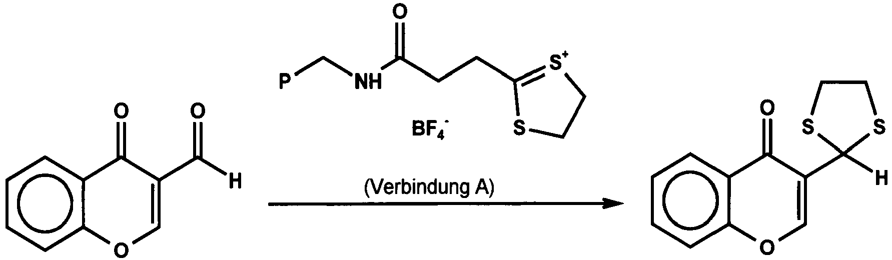

Es erfolgte ausschließlich die Umsetzung der Aldehydgruppe, die Ketogruppe wurde nicht umgesetzt.

Mittels der vorliegenden Erfindung wurden die folgenden geschützen Aldehyde und Ketone hergestellt:
- 2-Phenyl-1,3-dithian
- 2-(1,3-Dithian-2-yl)phenol
- 4-(1,3-Dithian-2-yl)benzonitril
- 2-(1,3-Dithian-2-yl)benzen-1,3-diol
- 2-(4-(Benzyloxy)phenyl)-1,3-dithian
- 2-(4-(Benzyloxy)phenyl)-1,3-dithiolan
- 4-(1,3-Dithian-2-yl)-2-methoxyphenol
- 4-(1,3-Dithiolan-2-yl)-2-methoxyphenol
- 2-([1,1'-Biphenyl]-4-yl)-1,3-dithian
- 2-(3-Methoxyphenyl)-1,3-dithian
- 2-(3-Methoxyphenyl)-1,3-dithiolan
- 2-Benzyl-1,3-dithian
- (E)-ethyl 3-(1,3-dithian-2-yl)acrylat
- (E)-ethyl 3-(1,3-dithiolan-2-yl)acrylat
- (E)-2-styryl-1,3-dithian
- (E)-2-(4-methoxystyryl)-1,3-dithian
- 3-(1,3-Dithiolan-2-yl)-4H-chromen-4-on
- 3-(1,3-Dithian-2-yl)-4H-chromen-4-on
- 3',4'-Dihydro-1'H-spiro[[1,3]dithian-2,2'-naphthalen]
- 1-Amino-2-(1,3-dithian-2-yl)anthracen-9,10(4aH,9aH)-dion
- 9-(tert-Butyl)-1,5-dithiaspiro[5.5]undecan

## Patentansprüche

1. Derivate von 1,3-Dithianen, 1,3-Dithiolanen der allgemeinen Formel 1a 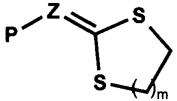 bzw. deren Salzen der allgemeinen Formel 1b 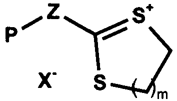 wobei
P = polymerer Träger,
m = 1 oder 2,
Z = ausgewählt aus der Gruppe bestehend aus den organischen Resten
-CH₂-Y-(CH₂)ₙ-CH₂-, -CH₂-Y-(CH₂)ₙ-CH=,
-CH₂-Y-CO-(CH₂)ₙ-CH₂-, -CH₂-Y-CO-(CH₂)ₙ-CH=, -CH₂-NH-C₆H₄-Y-(CH₂)ₙ-CH₂- oder -CH₂-NH-C₆H₄-Y-(CH₂)ₙ-CH=, bevorzugt ausgewählt aus der Gruppe bestehend aus den organischen Resten -CH₂-Y-(CH₂)ₙ-CH₂-, -CH₂-Y-(CH₂)ₙ-CH=, -CH₂-Y-CO-(CH₂)ₙ-CH₂- oder -CH₂-Y-CO-(CH₂)ₙ-CH=
wobei
Y = NH oder O,
n = 0 bis 10, bevorzugt 1 oder 2.
X = anorganisches oder organisches Anion.

2. Verbindungen nach Anspruch 1, **dadurch gekennzeichnet, dass** X ausgewählt wird aus der Gruppe bestehend aus Br, Cl⁻, Br₃⁻, ClO₄⁻, HCl₂⁻, BF₄⁻, CF₃SO₃⁻, insbesondere BF₄⁻, CF₃SO₃⁻.

3. Verbindungen nach Anspruch 1, **dadurch gekennzeichnet, dass** P ausgewählt wird aus der Gruppe bestehend aus Polystyrol, Polypropylen, Polyethylen, Polyethylenterephthalat, Silica, Cellulose und deren Mischungen, bevorzugt Polystyrol.

4. Verbindungen nach Anspruch 1, **dadurch gekennzeichnet, dass** sie ausgewählt werden aus der Gruppe bestehend aus den Verbindungen der folgenden Formeln 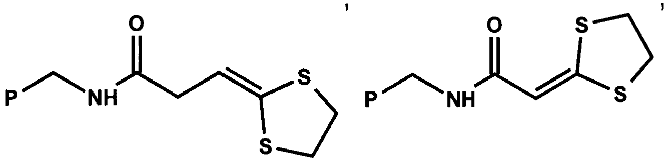 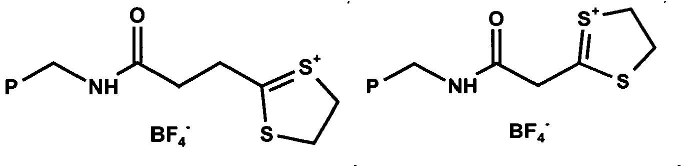 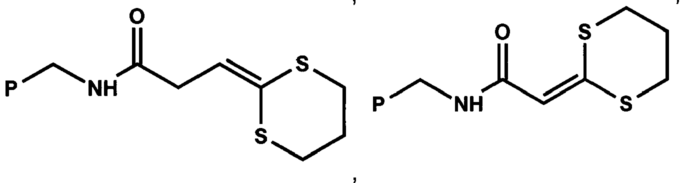 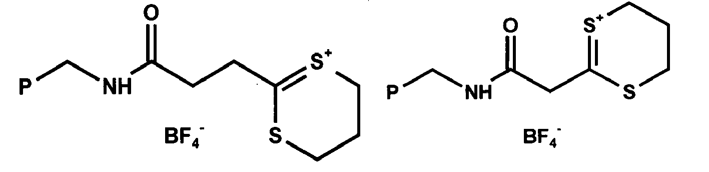 und Mischungen davon.

5. Verfahren zur Herstellung von Verbindungen gemäß einem der Ansprüche 1 bis 4 umfassend die Verfahrensschritte oder bestehend aus den Verfahrensschritten
a) Bereitstellen eines polymeren Trägers mit anhängigen funktionellen Gruppen
b) Umsetzung der funktionellen Gruppen des polymeren Trägers mit einem Kettenverlängerungsmittel,
c) gegebenenfalls Modifikation der funktionellen Gruppen des aus b) resultierenden kettenverlängerten Produktes,
d) Umsetzung des in b) bzw. c) erhaltenen Produktes mit gegebenenfalls substituierten aliphatischen oder aromatischen Dithioverbindungen mit einem oder zwei Kohlenstoffatomen zwischen den Schwefelatomen, bevorzugt aliphatischen Dithioverbindungen, insbesondere bevorzugt 1,2-Dithioethan oder 1,3-Dithiopropan,
e) optional Aufarbeitung des in Schritt d) erhaltenen Produktes
e1) zu den entsprechenden Dithiolanylium-Salzen/Dithianylium-Salzen (1b), bevorzugt durch Waschen unter Verwendung von Diethylether und THF,
oder
e2) zu Yliden-Dithiolanen/Dithianen (1a), bevorzugt durch Waschen mit anderen Lösemitteln als Diethylether/THF oder durch Zusatz von Base im Waschlösemittel.

6. Verfahren zur Herstellung von 1,3-Dithianen oder 1,3-Dithiolanen der allgemeinen Formel 1b 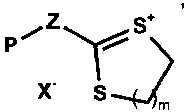 wobei die Variablen die oben genannten Bedeutungen haben, umfassend oder bestehend aus den folgenden Verfahrensschritten:
a) Bereitstellen von Polystyrol mit anhängigen -CH₂-NH₂-Gruppen,
b) Umsetzung mit Bernsteinsäureanhydrid in Dimethylformamid und/oder Triethylamin und in Gegenwart von 4-(Dimethylamino)-pyridin,
c) Umsetzung der resultierenden Carbonsäure mit Thionylchlorid zum Säurechlorid,
d) Umsetzung mit 1,2-Dithioethan oder 1,3-Dithiopropan in Gegenwart von HBF₄ in Diethylether,
e) optional Aufarbeitung des in Schritt d) erhaltenen Produktes durch Entfernen des Lösemittels und Waschen des zurückbleibenden Harzes, bevorzugt mit Diethylether und/oder THF und anschließendes Trocknen des Harzes.

7. Verfahren zur Herstellung von 1,3-Dithianen oder 1,3-Dithiolanen der allgemeinen Formel 1b 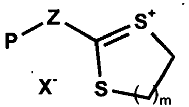 wobei die Variablen die oben genannten Bedeutungen haben, umfassend oder bestehend aus den folgenden Verfahrensschritten:
a) Bereitstellen von Polystyrol mit anhängigen -CH₂-NH₂-Gruppen,
b) Umsetzung mit Adipoyldichlorid in Dimethylformamid und/oder Triethylamin und in Gegenwart von 4-(Dimethylamino)-pyridin (DMAP),
d) Umsetzung mit 1,2-Dithioethan oder 1,3-Dithiopropan in Gegenwart von HBF₄ in Diethylether
e) optional Aufarbeitung des in Schritt d) erhaltenen Produktes durch Entfernen des Lösemittels und Waschen des zurückbleibenden Harzes, bevorzugt mit Diethylether und/oder THF und anschließendes Trocknen des Harzes.

8. Verwendung der Verbindungen gemäß einem der Ansprüche 1 bis 4 bzw. der gemäß Anspruch 5 bis 7 hergestellten Verbindungen, insbesondere gemäß der allgemeinen Formel 1b, als Reagenzien, bevorzugt für
die Einführung von Thioacetalschutzgruppen in Aldeyhde und/oder Ketone,
die Herstellung von Boranilen,
die Umsetzung von 1,3-Diketoverbindungen zu stabilen BF₂-Addukten, die Herstellung von Benzimidazolen
die Katalyse.

9. 2-(1,3-Dithian-2-yl)benzen-1,3-diol, 3',4'-Dihydro-1'H-spiro[[1,3]dithian-2,2'-naphthalen], 1-Amino-2-(1,3-dithian-2-yl)anthracen-9,10(4aH,9aH)-dion, (E)-Ethyl-3-(1,3-dithiolan-2-yl)acrylat und/oder (E)-Ethyl 3-(1,3-dithian-2-yl)acrylat.

## Claims

1. Derivatives of 1,3-dithianes, 1,3-dithiolanes of the general formular 1a 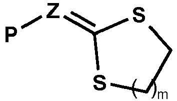 or the salts thereof of the general formular 1b, 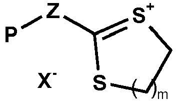 in which
P = polymeric carrier,
m = 1 or 2,
Z = selected from the group consisting of the organic radicals -CH₂-Y-(CH₂)ₙ-CH₂-, -CH₂-Y-(CH₂)ₙ-CH=, -CH₂-Y-CO-(CH₂)ₙ-CH₂-, -CH₂-Y-CO-(CH₂)ₙ-CH=, -CH₂-NH-C₆H₄-Y-(CH₂)ₙ-CH₂- or -CH₂-NH-C₆H₄-Y-(CH₂)ₙ-CH=,
preferably selected from the group consisting of the organic radicals -CH₂-Y-(CH₂)ₙ-CH₂-, -CH₂-Y-(CH₂)ₙ-CH=, -CH₂-Y-CO-(CH₂)ₙ-CH₂- or -CH₂-Y-CO-(CH₂)ₙ-CH=
in which
Y= NH or O,
n = 0 to 10, preferably 1 or 2,
X = inorganic or organic anion.

2. Compounds according to claim 1, **characterised in that** X is selected from the group consisting of Br⁻, Cl⁻, Br₃⁻, ClO₄⁻, HCl₂⁻, BF₄⁻, CF₃SO₃⁻, in particular BF₄⁻, CF₃SO₃⁻.

3. Compounds according to claim 1, **characterised in that** P is selected from the group consisting of polystyrene, polypropylene, polyethylene, polyethylene terephthalate, silica, cellulose and mixtures thereof, preferably polystyrene.

4. Compounds according to claim 1, **characterised in that** they are selected from the group consisting of the compounds of the following formulars 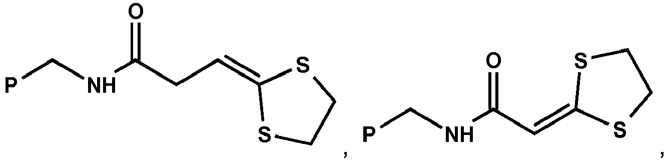 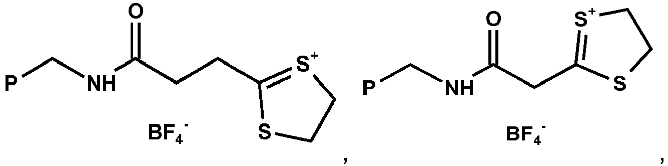 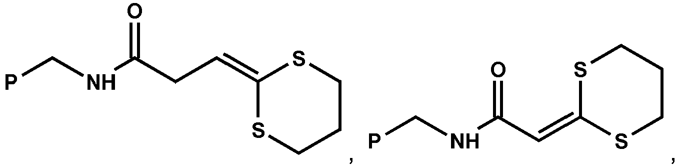 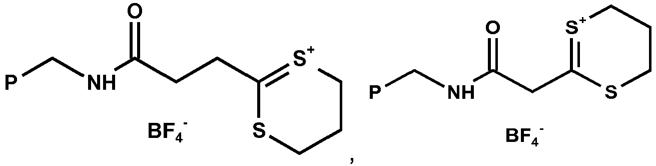 and mixtures thereof.

5. Process for the production of compounds according to any of the claims 1 to 4 comprising the steps of or consisting of the steps of
a) providing a polymeric carrier having pendant functional groups
b) reaction of the functional groups of the polymeric carrier with a chain extender,
c) optionally modification of the functional groups of the chain-extended product obtained from b),
d) reaction of the product obtained in b) or c) with unsubstituted or substituted aliphatic or aromatic dithio compounds having one or two carbon atoms between the sulfur atoms, preferably aliphatic dithio compounds, particularly preferably 1,2-dithioethane or 1,3-dithiopropane.
e) optionally working up the product obtained in step d)
e1) to form the corresponding dithiolanylium-salts/dithianylium-salts (1b), preferably by washing with using diethyl ether and THF,
or
e 2) to ylidene dithiolanes/dithianes (1a) ,preferably washing with other solvents as diethyl ether/THF or by addition of base in the wash solvent.

6. Process for the production of 1,3-dithianes or 1,3-dithiolanes of the general formular 1b), 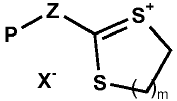 wherein the variables are as defined above, comprising or consisting of the following process steps:
a) providing of polystyrene having pendant -CH₂-NH₂ groups,
b) reaction with succinic anhydride in dimethylformamide and/or triethylamine and in the presence of 4-(dimethylamino)- pyridine,
c) reaction of the resulting carboxylic acid with thionyl chloride to the acid chloride,
d) reaction with 1,2-dithiolane or 1,3-dithiopropane in the presence of HBF₄ in diethyl ether.
e) optionally working up the product obtained in step d) by removing the solvent and washing the remaining resin , preferably with diethyl ether and/or THF , and subsequently drying the resin.

7. Process for the production of 1,3-dithianes or 1,3-dithiolanes of the general formular 1b), 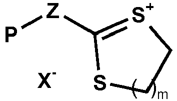 wherein the variables are as defined above, comprising or consisting of the following process steps:
a) Providing of polystyrene having pendant -CH₂-NH₂ groups,
b) reaction with adipoyl dichloride in dimethylformamide and/or triethylamine and in the presence of 4-(dimethylamino)- pyridine (DMAP),
d) reaction with 1,2-dithiolane or 1,3-ditiopropane in the presence of HBF in diethyl ether.
e) Optionally working up the product obtained in step d) by removing the solvent and washing the remaining resin , preferably with diethyl ether and/or THF , and subsequently drying the resin.

8. Use of the compounds according to any oft he claims 1 to 4 or of the compounds prepared according to claims 5 to 7, in particular according to the general formular 1b, as reagents , preferably for
the introduction of thioacetal protecting groups in aldehydes and/or ketones,
the preparation of boraniles,
the reaction of 1,3-diketo compounds to form stable BF₂ adducts ,
the preparation of benzimidazoles,
the catalysis.

9. 2-(1,3-dithiane-2-yl)benzene-1,3-diol, 3',4'-didydro-1'H-spiro[[1,3] dithiane-2,2'naphtalene], 1-amino-2-(1,3-dithian-2-yl)anthracene-9,10 (4aH,9aH)-dione, (E)-ethyl-3-(1,3-dithiolane-2-yl)acrylate and/or (E) ethyl 3-(1,3-dithiane-2-yl)acrylate.

## Revendications

1. Dérivés de 1,3-dithianes, 1,3-dithiolanes de la formule générale 1a 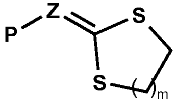 ou de leurs sels de la formule générale 1b, 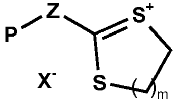 dans laquelle
P = un support polymère,
m = 1 ou 2,
Z = choisi du groupe consistant des résidus organiques -CH₂-Y-(CH₂)ₙ-CH₂-, -CH₂-Y-(CH₂)ₙ-CH=, -CH₂-Y-CO-(CH₂)ₙ-CH₂-, -CH₂-Y-CO-(CH₂)ₙ-CH=, -CH₂-NH-C₆H₄-Y-(CH₂)ₙ-CH₂- or -CH₂-NH-C₆H₄-Y-(CH₂)ₙ-CH=,
de préférence choisi du groupe consistant des résidus organiques -CH₂-Y-(CH₂)ₙ-CH₂-, -CH₂-Y-(CH₂)ₙ-CH=, -CH₂-Y-CO-(CH₂)ₙ-CH₂- or -CH₂-Y-CO-(CH₂)ₙ-CH=
dans laquelle
Y= NH or O,
n = 0 to 10, de préférence 1 ou 2,
X = un anion inorganique ou organique.

2. Compositions selon la revendication 1, **caractérisées en ce que** X est choisi du groupe consistant de Br⁻, Cl⁻, Br₃⁻, ClO₄⁻, HCl₂⁻, BF₄⁻, CF₃SO₃⁻, particuliairement BF₄⁻, CF₃SO₃⁻.

3. Compositions selon la revendication 1, **caractérisées en ce que** P est choisi du groupe consistant de polystyrène, polypropylène, polyéthylène, téréphthalate de polyéthylène , silice, cellulose et leurs mixtures, de préférénce polystyrène.

4. Compositions selon la revendication 1, **caractérisées en ce qu'**elles sont choisies du groupe consistant des compositions des formules suivantes 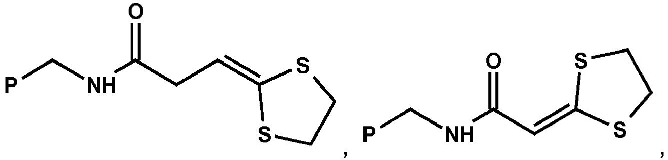 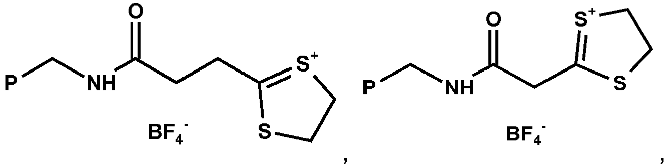 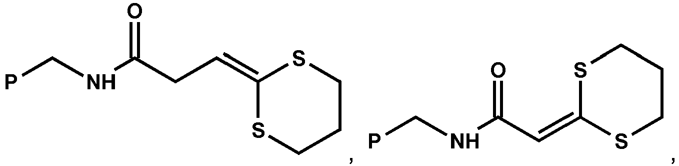 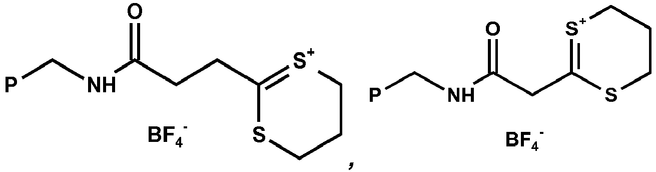 et de leurs mixtures.

5. Procédé pour la production des compositions selon l'une quelconque des revendications 1 to 4 comprenant des étapes ou consistant d' étapes de procédé de
a) fournir un support polymère avec des groupes attachés functionnels
b) réaction des groupes functionnels du support polymère avec un moyen d' extension de chaîne,
c) le cas échéant modification des groupes functionnels du produit résultant d'extension de chaîne d'étape b),
d) réaction du produit obtenu des étapes b) ou c) avec des compositions dithio, non substitutés ou substitutés aliphatiques ou aromatiques avec un ou deux atomes de carbon entre des atomes sulfuriques , de préférence des compositions dithio aliphatiques dithio, plus particulièrement 1,2-dithioéthane ou 1,3-dithiopropane,
e) le cas échéant traiter le produit obtenu de l'étape d)
e1) pour former les sels correspondants de dithiolanylium/dithianylium (1b), de préférence en laver avec éther de diéthyl et THF, ou
e 2) à ylidène de dithiolanes/dithianes (1a) , de préférence laver avec d autres solvants comme éther de diéthyl /THF ou par addition de base au solvant à laver.

6. Procédé de production de 1,3-dithianes ou 1,3-dithiolanes de la formule générale 1b), 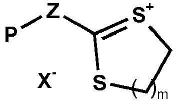 dans laquelle les variables sont comme définies au-dessus, comprenant ou consistant d' étapes de:
a) Fournir de polystyrène avec des groupes attachés -CH₂-NH₂,
b) réaction avec anhydride succinique en diméthylformamide et/ou triéthylamine et en présence de 4-(diméthylamino)-pyridine,
c) réaction de l'acide carboxylique résultant avec chloride de thionyl à l'acide de chloride,
d) réaction avec 1,2-dithiolane ou 1,3-dithiopropane en la présence de HBF₄ en diéthyl éther.
e) le cas échéant traiter le produit obtenu de l'étape d) en éloigner le solvant et laver la résine restante, de préférance avec diéthyl éther et/ou THF, et ensuite sècher la résine.

7. Procédé de production de 1,3-dithianes ou 1,3-dithiolanes de la formule générale 1b), 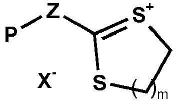 dans laquelle les variables sont définies comme ci-dessus, comprenant ou consistant d' étapes de procédé suivantes:
a) Fournir de polystyrène avec des groupes attachés -CH₂-NH₂,
b) réaction avec adipoyl dichloride en diméthylformamide et/ou triéthylamine en présence de 4-(diméthylamino)- pyridine (DMAP),
d) réaction avec 1,2-dithiolane ou 1,3-dithiopropane en présence de HBF en diéthyl éther.
e) le cas échéant traiter le produit obtenu en l'étape d) en éloigner le solvant et laver la résine restante, de préférance avec diéthyl éther et/ou THF, and après sécher la résine.

8. Utilisation des compositions selon l'une quelconque des revendications 1 à 4 ou des compositions préparées selon les revendications 5 à 7, particulièrement selon la formule générale 1b, comme réactifs, de préférence pour
l'introduction des groupes protectifs de thioacétal en aldéhydes et/ou kétones,
la préparation de boraniles,
la réaction de compositions 1,3-diketo pour former des adduits stables BF₂,
la préparation de benzimidazoles,
la catalyse.

9. 2-(1,3-dithiane-2-yl) benzène-1,3-diol, 3',4'-didydro-1'H-spiro[[1,3] dithiane 2,2'naphtalène], 1-amino-2-(1,3-dithiane-2-yl)anthracène-9,10 (4aH,9aH)-dione, (E)-éthyl-3-(1,3-dithiolane-2-yl)acrylate et/ou (E) éthyl 3-(1,3-dithiane-2-yl)acrylate.
